# EUROPEAN PATENT SPECIFICATION

(11) **EP 1 909 106 B1**
(45) Date of publication and mention of the grant of the patent: **02.01.2013**
(21) Application number: 07023170.9
(22) Date of filing: 12.09.2002
(51) Int. Cl.: G01N 33/84, G01N 33/90, G01N 33/68

(54) **Differential diagnosis of disorders of iron metabolism by means of four parameters and recommendations for the treatment of these disorders of iron metabolism**
Differenzialdiagnose von Eisenstoffwechselstörungen mittels vier Parameter und Empfehlungen für die Behandlung dieser Eisenstoffwechselstörungen
Diagnostic différentiel de troubles du métabolisme du fer à l'aide de quatre paramètres et recommandation pour le traitement de ces troubles du métabolisme du fer

(30) Priority: 14.09.2001 US 322526 P
(43) Date of publication of application: 09.04.2008
(62) Divisional of application: 02777081.7
(73) Proprietor: F.Hoffmann-La Roche AG, 4070 Basel (CH); Roche Diagnostics GmbH, 68305 Mannheim (DE)
(72) Inventor: Thomas, Lothar, 60489 Frankfurt (DE); Lehmann, Paul, 67549 Worms (DE); Röddiger, Ralf, 69517 Gorxheimertal (DE)
(74) Representative: Dey, Michael

(56) References cited:
- WO-A1-97/09996
- WO-A2-99/07401
- BOVY CHRISTOPHE ET AL: "Factors determining the percentage of hypochromic red blood cells in hemodialysis patients." KIDNEY INTERNATIONAL, vol. 56, no. 3, 1999, pages 1113-1119, XP002247457 ISSN: 0085-2538
- TESSITORE NICOLA ET AL: "The role of iron status markers in predicting response to intravenous iron in haemodialysis patients on maintenance erythropoietin." NEPHROLOGY DIALYSIS TRANSPLANTATION, vol. 16, no. 7, July 2001 (2001-07), pages 1416-1423, XP002247458 ISSN: 0931-0509
- THOMAS CHRISTIAN ET AL: "Biochemical markers and hematologic indices in the diagnosis of functional iron deficiency." CLINICAL CHEMISTRY. UNITED STATES JUL 2002, vol. 48, no. 7, July 2002 (2002-07), pages 1066-1076, XP002247459 ISSN: 0009-9147
- DATABASE MEDLINE US NATIONAL LIBRARY OF MEDICINE (NLM), BETHESDA, MD, US; 2002, HASEGAWA MIDORI ET AL: "[Evaluation of reticulocyte hemoglobin content, percentage of hypochromic red blood cells, and ratio of serum transferrin receptor level/serum iron level as markers of iron-deficiency erythropoiesis in patients undergoing hemodialysis]" XP002247472 & NIPPON JINZO GAKKAI SHI. JAPAN 2002, vol. 44, no. 5, 2002, pages 453-463,
- PUNNONEN KARI ET AL: "Serum transferrin receptor and its ratio to serum ferritin in the diagnosis of iron deficiency" BLOOD, vol. 89, no. 3, 1997, pages 1052-1057, XP002539616 ISSN: 0006-4971
- BRUGNARA C ET AL: "RETICULOCYTE HEMOGLOBIN CONTENT TO DIAGNOSE IRON DEFICIENCY IN CHILDREN" JAMA THE JOURNAL OF THE AMERICAN MEDICAL ASSOCIATION, AMERICAN MEDICAL ASSOCIATION, US, vol. 281, no. 23, 1 June 1999 (1999-06-01) , pages 2225-2230, XP009078666 ISSN: 0098-7484
- FISHBANE S ET AL: "RETICULOCYTE HEMOGLOBIN CONTENT IN THE EVALUATION OF IRON STATUS OF HEMODIALYSIS PATIENTS" KIDNEY INTERNATIONAL, NATURE PUBLISHING GROUP, LONDON, GB, vol. 52, no. 1, 1 July 1997 (1997-07-01), pages 217-222, XP009078669 ISSN: 0085-2538
- BRUGNARA C ET AL: "RETICULOCYTE HEMOGLOBIN: AN INTEGRATED PARAMETER FOR EVALUATION OF ERYTHROPOIETIC ACTIVITY" AMERICAN JOURNAL OF CLINICAL PATHOLOGY, AMERICAN SOCIETY FOR CLINICAL PATHOLOGY, US, vol. 108, no. 2, 1 August 1997 (1997-08-01), pages 133-142, XP009078670 ISSN: 0002-9173
- ALEXANDER H D ET AL: "Red cell indices as predictors of iron depletion in blood donors.", CLINICAL AND LABORATORY HAEMATOLOGY OCT 2000 LNKD- PUBMED:11122264, vol. 22, no. 5, October 2000 (2000-10), pages 253-258, XP007920136, ISSN: 0141-9854

## Description

The invention concerns a method for detecting disorders of iron metabolism and in particular the differential diagnosis of disorders of iron metabolism by means of three independent parameters. The differential diagnosis can be used to classify disorders of iron metabolism and to recommend the required treatment and to monitor the progress and response to treatment.

Iron as a component of haemoglobin and the cell haemins is one of the most important biocatalysts in the human organism. Disorders of iron metabolism and in particular iron deficiency and perturbations of iron distribution and utilization in chronic general illnesses are among the most frequently overlooked or misinterpreted diseases. One of the main reasons for this is that the determination of transport iron in the serum or plasma which is used in conventional diagnostics does not allow a representative estimation of the total body iron stores due to short-term variations.

The ability to precisely determine the iron storage protein ferritin in plasma provided a method for determining the total body iron stores and thus allowed a more rapid and reliable diagnosis especially of iron deficiency states. Ferritin is an indicator of the amount of storage iron. The soluble transferrin receptor (sTfR) indicates the iron requirements of the cell and erythropoiesis activity. The sTfR/log ferritin index is a measure of the depletion of the iron stores and of the functional iron compartments. In chronic inflammatory diseases such as in infections and especially tumour diseases, iron is redistributed with a relative overload of the iron stores accompanied by a relative deficiency of iron supply to the erythropoietic cells.

Due to the very limited capacity to absorb iron, the iron requirements can only be met by recycling functional iron. It is stored in the form of ferritin and haemosiderin. Each cell is able to take up a surfeit of iron by synthesizing ferritin and the basic mechanisms for this are identical in all types of cells. The transferrin-iron³⁺ complex is bound to the transferrin receptor of the cell membrane. The uptake of iron can be regulated by the transferrin receptor expression. In addition iron induces the synthesis of apoferritin. Hence in the majority of metabolic situations a representative proportion of the synthesized ferritin is released into the blood plasma.

However, even if the above-mentioned parameters are employed, it is not in practice possible or very difficult to routinely determine and differentiate between various iron states.

Bovy et al. (Kidney International, 1999, 56(3):1113-1119) relates to factors that determine the percentage of hypochromic red blood cells (%HYPO) in haemodyalysis patients. It was shown that the variation of %HYPO is essentially associated with the combined changes in sTfR, CRP and EPO dosage.

Thomas and Thomas (Clinical Chemistry, 2002, 48(7):1066-1076) relates to the use of biochemical markers and haematologic indices, in particular CHr, sTfR, Ferritin and CRP, in the diagnosis of functional iron deficiency.

Punnonen et al. (Blood, 1997, 89(3):1052-1057) describes a study for evaluating the diagnostic efficiency of measurements of the parameter serum ferritin and serum transferrin receptor in the diagnosis of iron depletion.

WO 97/09996 relates to a pharmaceutical combination preparation for the treatment of amaemias or haemodialysis patients. Determination of the diagnostic parameters transferrin receptor (TfR) and ferritin as well as acute phase parameter CRP is disclosed.

WO 99/07401 relates to pharmaceutical combination preparations for the treatment of rheumatic diseases. Determination of the diagnostic parameters transferrin receptor (TfR) and ferritin as well as actue phase parameter CRP is disclosed.

Alexander et al. (Clinical and Laboratory Haematology, 2000, 22(5):253-258) describes a study investigating red cell indices such as MCV and MCH as predictors of iron depletion in blood donors.

It was an object of the present invention to provide a method which enables the reliable detection of disorders of iron metabolism in a simple manner.

In vitro method for determining the iron status and in particular for detecting disorders of iron metabolism comprising the determination of
(i) a parameter which allows a determination of the total body iron stores,
(ii) a parameter which allows a determination of the erythropoietic maturation process and/or its activity,
(iii) a parameter which allows a determination of unspecific disorders of iron metabolism, wherein the parameter is a biochemical parameter, and
(iv) a haematological parameter,
wherein ferritin is determined as parameter (i), soluble transferrin receptor (sTfR) is determined as parameter (ii) CRP is determined as parameter (iii), and MCH is determined as parameter (iv), wherein MCH is the average hemoglobin content of an erythrocyte cell, characterized in that
differential diagnosis is carried out.

Hence the invention concerns the differential diagnosis of disorders of iron metabolism by means of four independent parameters.

The determination of the total body iron stores can for example be carried out by measuring the parameters erythrocyte ferritin, zinc protoporphyrin, haemoglobin, myoglobin, transferrin and transferrin saturation, ferritin, haemosiderin or/and the enzymes catalase, peroxidase or/and cytochrome. A determination of the concentration or activities of these parameters enables a determination of the total body iron stores. According to the invention, ferritin is used as the parameter (i).

The erythropoietic maturation process and/or the erythropoietic activity can for example be ascertained or determined using erythrocyte indices, reticulocyte indices, FS-e (forward scatter erythrocytes) and/or the soluble transferrin receptor (sTfR). The amount or concentration of soluble transferrin receptor (sTfR) is determined as parameter (ii) in the method according to the invention and used as a parameter for the erythropoietic maturation process or its activity.

Biochemical parameters as well as haematological parameters can be used as a parameter- for determining unspecific disorders of iron metabolism. Acute phase proteins and regulators of acute phase protein synthesis are preferably used as biochemical parameters whereas disorders of reticulocyte synthesis are preferably used as haematological parameters. Examples of acute phase proteins whose amount or concentration is determined in order to determine unspecific disorders of iron metabolism comprise C-reactive protein (CRP), serum amyloid A (SAA), α₁-anti-chymotrypsin, acidic α₁-glycoprotein, α₁-antitrypsin, haptoglobin, fibrinogen, complement component C3, complement component C4 or/and coeruloplasmin. Examples of regulators of acute phase protein synthesis are interleukin 6 (IL-6), leukaemia inhibiting factor (LIF), oncostatin M, interleukin 11 (IL-11), ciliary neurotropic factor (CNTF), interleukin 1α(IL-1α), interleukin 1ß (IL-1ß), tumour necrosis factor-α (TNFα), tumour necrosis factor-ß (TNFß), insulin, fibroblast growth factor (FGF), hepatocyte growth factor, transgrowth factor ß (TGFß) or/and interferon.

Disorders of reticulocyte synthesis such as CH₂, reticulocyte count, Hb content of reticulocytes (CHr), IRF (immature reticulocyte fraction) new RBC and reticulocyte fluorescence parameters and/or FS-r (forward scatter reticulocytes) are haematological parameters that can be used. According to the invention CPR is used as parameter (iii).

It was found that rapid and reliable information on the iron status of patients can be obtained by combining three independent parameters. In particular it was found that biochemical or haematological markers and in particular inflammatory markers which are unspecific as such, can be used in an appropriate combination with other parameters to determine the iron status.

In particular the method according to the invention allows a classification of the iron status and in particular of disorders of iron metabolism.

The combination of three independent parameters enables a routine differentiation between normal iron status, iron deficiency, iron distribution disorders and/or iron overloading. Differentiation between normal iron status and iron overloading can be achieved. In addition a differentiation between the status of iron deficiency and iron distribution disorders can be achieved. Perturbations of iron distribution can lead to chronic diseases such as rheumatism, asthma or tumours and hence an early detection of iron distribution disorders is of particular importance.

In a particularly preferred embodiment the iron status determined by the method according to the invention is classified in one of the following groups:
(A) iron distribution disorder or/and iron utilization disorder with acute phase reaction,
(B) iron overloading,
(C) normal iron status, and
(D) deficiency of storage iron.

The evaluation of the determined parameters can be preferably assisted by a computer for example by use of an anaemia program. Furthermore the determined measurements are preferably represented graphically in the form of diagrams in order to easily assign the measuring ranges to the various iron states. For example parameter (iii) can be plotted on the ordinate and the ratio of parameter (ii) to parameter (i) can be plotted on the abscissa. This results in various measuring ranges (fields in the diagram) for the various iron states and iron overloading can be distinguished from a normal iron status, and a normal iron status can be distinguished from iron deficiency and also from iron distribution disturbances such as tumour anaemia, chronic anaemia, rheumatoid arthritis or renal anaemia.

The method according to the invention can also be used to specify in a simple manner the treatment required for the respective patient depending on the determined iron status. Thus for example erythropoietin (EPO) therapy is indicated for a classification in group (A), blood letting is indicated for a classification in group (B), no therapy is indicated for a classification in group (C) and iron substitution is indicated when classified in group (D). These therapeutic recommendations are based on the fact that erythropoiesis is mainly regulated by the growth factor EPO and by iron, and the various types of iron metabolism disorders require different treatments that can be determined by the method according to the invention. An iron deficiency leads in particular to a deficit in haemoglobin formation, to hypochromic mycrocytes/anulocytes and thus to anaemias which are manifested as iron deficiency and chronic bleeding. Deficiency of erythropoietin (EPO) results in a reduced proliferation and thus to anaemias that manifest themselves as iron distribution disturbances, acute phase conditions, infections, chronic inflammation, tumour anaemias and renal anaemias.

In addition to the treatment of disorders of iron metabolism, the method according to the invention also allows observation or/and monitoring of the progress and response to treatment and thus ensures an optimal use of EPO or iron preparations (e.g. oral or parenteral iron preparations) in individual patients.

Depending on the selected characteristic values of the above mentioned parameters, the method also allows a sex-specific discrimination or differentiation of the individual iron status in which the normal values or cut-off values can then be established for each sex (male or female).

Surprisingly, it was found that chronic diseases, even in very early stages, result in a classification in group (A). Thus, chronic diseases and chronic inflammatory diseases can be diagnosed with the method according to the invention. In particular, diseases such as renal insufficiency, malignancies, rheumatoid arthritis, diabetes, heart failure, cardiovascular diseases, thrombosis, neurogenerative diseases or impaired pregnancies can be identified, and respective treatments can be indicated.

Group (B) indicating iron overloading includes haemochromatosis such as sickle cell anemia or HFE gene modifications. sTfR is determined as parameter (ii). It was found that the soluble transferrin receptor (sTfR) is a parameter for the following three types of iron status:
(a) haemoglobin synthesis rate,
(b) repletion status of the iron stores (ferritin) and
(c) non-ferritin iron deposition (disturbance in distribution, iron deposition).

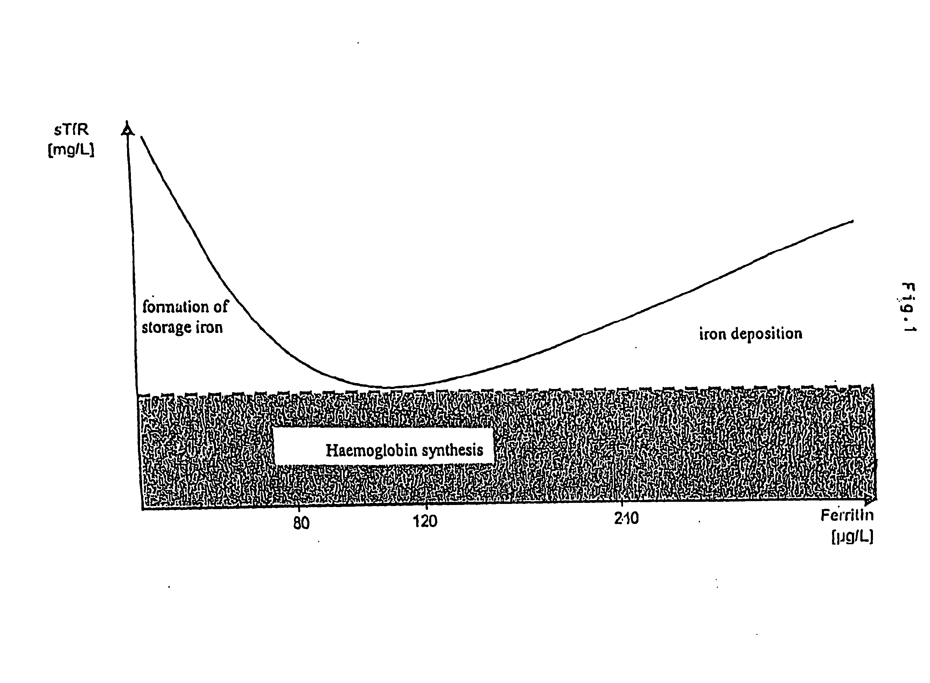

In addition the ferritin content is determined as parameter (i). A combination of sTfR and ferritin yields information on the depletion of iron stores, haemoglobin synthesis and iron deposition as shown in figure 1.

These two parameters for determining the iron status i.e. sTfR and ferritin are combined with the biochemical marker CRP.

This combination can serve in particular as diagnostic markers for chronic anaemias (ACD) in rheumatic diseases.

In order to efficiently differentiate between the anaemias, the classification is carried out by calculating the ratio of sTfR/log ferritin. It is standardized on the basis of the CRP value. For the graphic representation the ratio of sTfR/log ferritin is plotted on the X axis and the CRP value is plotted on the Y axis. This results in the following classification into the various types of iron status shown in table 1:

**Table 1: Differentiation and treatment recommendations for various anaemias using sTfR, ferritin and CRP values**

| Quadrant* | Ferritin [µg/L] | sTfR [mg/L] | sTfR log ferritin | CRP [mg/L] | Comments |
|---|---|---|---|---|---|
| A | > 30♂ | high↑ (measure of erythropoietic activity) | < 3.4♂ | > 5 | disturbances of iron distribution |
| | > 15♀ | | < 3.7♀ | > 5 | disturbances of iron utilization with acute phase reaction (EPO therapy) |
| B | > 400♂ | < 5 | < 0.9♂ | < 5 | iron overloading (blood letting therapy) |
| | > 150♀ | > 4.4 | < 0.9♀ | < 5 | |
| C | 30-400♂ | > 5 | < 3.4♂ | < 5 | normal iron status, no acute phase reaction |
| | 15-150♀ | > 4.4 | < 3.7♀ | < 5 | |
| D | < 30♂ | > 5 | > 3.4♂ | < 5 | deficiency of stored iron, no acute phase reaction (iron substitution) |
| | < 15♀ | > 4.4 | > 3.4♀ | < 5 | |
| | < 30♂ | very high↑↑ (measure of iron requirements of the cells | > 3.4♂ | > 5 | deficiency of stored iron with acute phase reaction (iron substitution) |
| | < 15♀ | | > 3.7♀ | | |

| | | | | | |
|---|---|---|---|---|---|
| * see Fig.2 | | | | | |

The cut-off values shown in table 1 are derived from the reference ranges for women (premenopausal) for sTfR of 1.9 to 4.4 mg/l, ferritin of 15 to 150 µg/l and CRP of < 5 mg/l and for men for sTfR of 2.2 to 5.0 mg/l, ferritin of 30 to 400 µg/l and CRP of < 5 mg/l. When this is represented graphically results in four quadrants which are defined by the cut-off values for CRP of 5 mg/l and for the ratios sTfR/log ferritin of 3.4 (men) and 3.7 (women) and 0.9. This enables anaemias which are caused by perturbations of iron distribution (A), iron deficiency (D) and iron overloading (B) to be distinguished from the normal iron status (C).
In a particularly advantageous method the differential diagnosis of the important disorders of iron metabolism is assisted by a software program which enables a mathematical linkage of the three above-mentioned independent parameters. The following independent parameters are used:
(i) ferritin as a parameter that allows an estimate of the actual body iron stores (depot iron),
(ii) sTfR as a parameter which allows an estimation of the erythropoietic activity (functional iron) and
(iii) CRP as a parameter for the diagnosis of unspecific disorders of iron metabolism
which are caused for example by inflammatory processes.

In this manner the method enables disorders of iron metabolism to be described by using the iron storage protein ferritin and the soluble transferrin receptor as an indicator for the iron requirements of the cells. In addition the determination of the soluble transferrin receptor enables an estimate of the erythropoietic activity. CRP acts as an indicator of a persistent acute phase reaction. The correlation between CRP and the ratio of sTfR/log ferritin allows an efficient differential diagnosis of anaemias such as iron deficiency, iron distribution disorders and iron overloading from normal iron status. The differential diagnosis can be further simplified for the user by a computer-aided evaluation program.

A latex-enhanced immunoturbidimetric assay can for example be used to determine the soluble transferrin receptor for use in a method in combination with the determination of ferritin and CRP. The values for sTfR stated herein in connection with methods using sTfR, ferritin and CRP refer to values measured with latex-enhanced immunoturbidimetric assays. The latex-enhanced immunoturbidimetric assay have an adequately sensitive measuring accuracy for detecting the relatively low concentrations of soluble transferrin receptor in the blood plasma (< 10 mg/l, or < 100 nmol/l). Since international reference methods and reference reparations are not yet available for sTfR, reference intervals on the COBAS INTEGRAR and Roche/Hitachi were determined for the test described herein and the sTfR reference range was 2.2 to 5.0 (2.5 to 97.5 percentile) for men and 1.9 to 4.4 for women.

The cut-off value for sTfR/log ferritin which discriminates between the iron status of iron overloading and normal iron status is 0.7 to 1.4, in particular 0.8 to 1.0 and most preferably 0.9. The cut-off value with which iron deficiency can be distinguished from iron distribution disorders and normal iron status is preferably 3.0 to 4.0, more preferably 3.4 to 3.7 and most preferably at about 3.4 for men and at about 3.7 for women. Calibration to determine these values was made as described by S.Kolbe-Busch et al., Clin.Chem.Lab.Med.40(5) (2002), 529-536. sTFR from placenta was used as standard thereby. The cut-off value for CRP above which an acute phase reaction is defined, is preferably at about 1 to 10 mg/l, more preferably at 4 to 6 mg/l and in particular at about 5 mg/l.

A haematological parameter is for example the proportion of hypochromic red blood cells (HRC%) or the haemoglobin content of reticulocytes (CHr). It was found that these parameters are indicators for functional iron deficiency. These parameters can be used in addition to biochemical markers such as ferritin, transferrin saturation (TfS) and transferrin receptor (TfR) to identify an iron deficiency (ID).

The haematological parameters show rapidly and directly any change in erythropoietic activities.

Non-anaemic patients without APR (acute phase reaction) have a CHr of ≥ 28 pg and HCR of ≤ 5 %. Patients with a CHr of < 28 pg or a HCR of > 5 % were classified as functionally iron deficient. Serum ferritin, TfS, TfR and the calculated parameters TfR-F index (ratio TfR/log ferritin) and Tf-Tf-R product enable a reliable diagnosis of iron deficiency in comparison with HCR % and CHr in patients without APR. In the case of anaemias without APR which are often observed in infections, inflammation or tumours, the diagnostic effectiveness of the said biochemical markers ferritin and transferrin receptor is often inadequate. A combination of these biochemical markers with haematological markers such as CHr considerably improves the results. When CHr is plotted against the TfR-F index or against the Tf-TfR product, it is possible to classify anaemias in patients with and without APR inter alia into the following categories: no functional iron deficiency, functional iron deficiency combined with depleted iron stores and functional iron deficiency combined with replete iron stores.

This method enables an identification of iron deficiency and a distinction of iron deficiency from other disorders of iron metabolism, in particular so-called anaemias, from chronic diseases (ACD) which accompany infections, inflammation or tumours. ACD is characterized by an inadequate erythropoietin production, inhibition of the proliferation of erythrocyte precursor cells in the bone marrow and disturbances of iron utilization. As in iron deficiency anaemia (IDA), functional iron deficiency in ACD is one of the main distinguishing factors from erythropoiesis. It is defined as an imbalance between iron requirements in the erythroid bone marrow and iron supply which is not sufficient to ensure a normal haemoglobination of red blood cells. This results in a reduced haemoglobin concentration in reticulocytes and erythrocytes. In IDA the iron supply depends on the content of the iron stores, and in the case of ACD on the rate of its mobilization. In ACD a functional iron deficiency can occur even in the presence of large iron stores if the iron release is impaired.
The diagnosis of a functional iron deficiency is important for the correct treatment of the patients. However, in practice it is often only possible to classify the patients as iron deficient, non-iron deficient or potentially iron-deficient. The third group of patients which are typically those with an acute phase reaction (APR) or a cancer related anaemia (CRA) have previously required an examination of their bone marrow in order to determine the type of disease.

Usually biochemical markers of iron metabolism are used such as serum or plasma iron, transferrin, % transferrin saturation (TfS), ferritin and serum-circulating transferrin receptor (TfR). The diagnosis of IDA is based on the presence of anaemia and morphological features of erythrocytes (hyperchromia, mycrocytosis) in conjunction with a low serum ferritin and a reduced transferrin saturation. The diagnosis of ID in conjunction with normal serum ferritin contents may, however, be difficult in the case of ACD. Ferritin is an acute phase reactant, transferrin is a negative acute phase reactant and the concentration of both proteins is influenced by various conditions. An increase in TfR which is a useful indicator for iron deficiency, can also occur in patients with an increase in the number of red precursor cells in the bone marrow. Due to these difficulties it is necessary to provide clinical laboratory tests which measure the functional availability of iron for haemoglobin synthesis especially in the red blood cells and their precursors.

A marker which can be used to assess the functional iron status, is the measurement of the proportion of hypochromic red cells (HRC %). Due to the life time of erythrocytes of about 120 days, HCR % integrates information over a long period and is thus a late indicator for iron-limited erythropoiesis. A value for HCR of < 10 % in conjunction with low serum ferritin indicates that the iron supply for erythropoiesis is sufficient to enable a normal haemoglobination of red cells.

The cellular haemoglobin content of reticulocytes (CHr) is an early marker for functional iron deficiencies since reticulocytes exist in the circulation for only 1 to 2 days. The utility of this index for monitoring the erythropoietic function in order to assess the iron status, to diagnose an iron deficiency and to diagnose and treat various haematological diseases is known.

A combination of the haematological indices HRC % or/and CHr with biochemical markers is described.

Using the 2.5 and 97.5 percentiles of the control group, the following cut-offs were determined 3 to 7 %, in particular 4 to 6 % and most preferably about 5 % for HCR and 25 to 30 pg, in particular 27 to 29 pg and particularly preferably about 28 pg for CHr. The iron status can preferably be classified using a diagnostic plot in which CHr is plotted against TfR-F or against Tf-Tf-R. In this manner the iron status can be divided into various categories and in particular four categories i.e. normal iron status, iron deficiency (CRP normal), iron deficiency (CRP increased) and iron distribution disorder.

The invention relates to a method for determining the iron status and, in particular, for detecting disorders of iron metabolism comprising the determination of
(i) a parameter which allows determination of the total body iron stores: ferritin,
(ii) a parameter which allows determination of the erythropoietic maturation process and/or its activity: sTfR,
(iii) a parameter which allows determination of unspecific disorders of iron
   metabolism, the biochemical parameter CRP, and
(iv) a haematological parameter: MCH.

In this method group (A) concerning patients who probably have disturbances of iron distribution (acute deficiency of functional iron) can be further divided in two groups. In particular, patients having no acute deficit of functional iron can be distinguished from patients actually having functional iron deficiency or disturbance of iron distribution. MCH can be determined from blood count. MCH is the average hemoglobin content of an erythrocyte cell and is reduced, if an acute deficiency of functional iron and thus a disturbance of iron distribution occurs. Therefore, MCH is used to distinguish a deficiency of functional iron from other other conditions. 28 pg/cell is to be regarded as a limiting value of MCH, whereby no acute deficiency of functional iron is the case for values above that value and deficiency of functional iron is diagnosed, if values are lower.

In a preferred embodiment, for example, CRP will be determined competitively and the other two parameters by using a sandwich assay.
Figure 1 shows that the soluble transferrin receptor (sTfR) is a parameter for three types of iron status. For (A) (iron distribution disturbance) this means Hb synthesis plus iron deposition, for (B) (iron overloading) Hb synthesis plus iron deposition, for (C) (normal iron status) Hb synthesis and for (D) (iron deficiency) Hb synthesis plus storage iron.

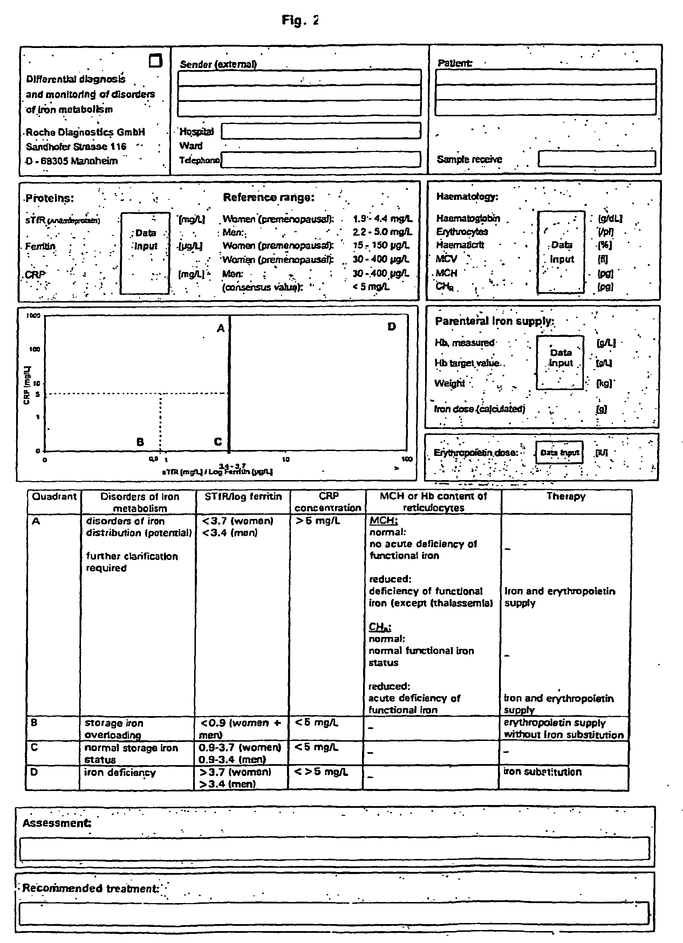

Figure 2 shows an example of an input for an anaemia program, the classification of the four quadrants A, B, C, D in a diagram of CRP against sTfR/log ferritin, the classification of the squares and the treatment recommended in each case.
Figure 3 shows the classification used in a combination of haematological and biochemical markers.

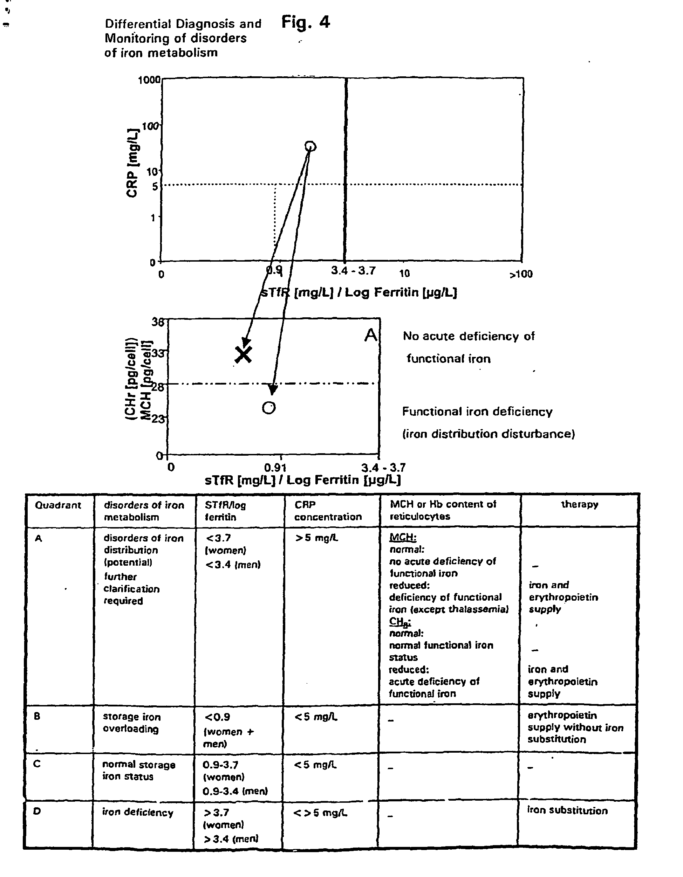

Figure 4 shows a method according to the invention, wherein group (A) is further divided by determination of the haematological parameter, MCH, Determination of CHr as a haematological parameter is an alternative not part of the invention.

### Comparative Example 1

163 patients were examined using the parameters CRP and sTfR/log ferritin and classified according to the results obtained as normal iron status, iron deficiency, iron distribution disturbance or iron overloading. The combined determination of the three parameters sTfR, ferritin and CRP proved to be highly suitable for differential diagnosis.

### Comparative Example 2

.373 patients were examined using a combination of haematological parameters and biochemical parameters and classified into four groups. Group N is the control group and contained non-anaemic patients without APR. Group A consists of anaemic patients without APR. Group AA contains anaemic patients with APR in combination with CRA, ACD or an acute infectious or inflammatory disease. The patient group NA contains non-anaemic patients with APR.

Ferritin was determined on a Cobascore analyzer from Roche Diagnostics, Mannheim, Germany and the reference range was determined as 20 to 150 µg/l for women and 20 to 350 µg/l for men. TfR was determined in each sample using commercial assays. The analytical principle of the assay (Dadebehring, Marburg) is based on microagglutination of latex particles which are coated with a monoclonal anti-TfR antibody. In this manner a latex-enhanced nephelometric test is carried out. The reference range (2.5 to 97.5 percentile) was 0.4 to 1.8 mg/l.

TfS was calculated using the formula TfS (%) = Fe (µg/l) x 7.09/Tf (g/l).

In order to determine disorders of iron metabolism CHr and HRC % were determined as indicators of an iron deficient erythropoiesis as a plot against the TfR-F index. The following results were obtained for the individual patient groups.

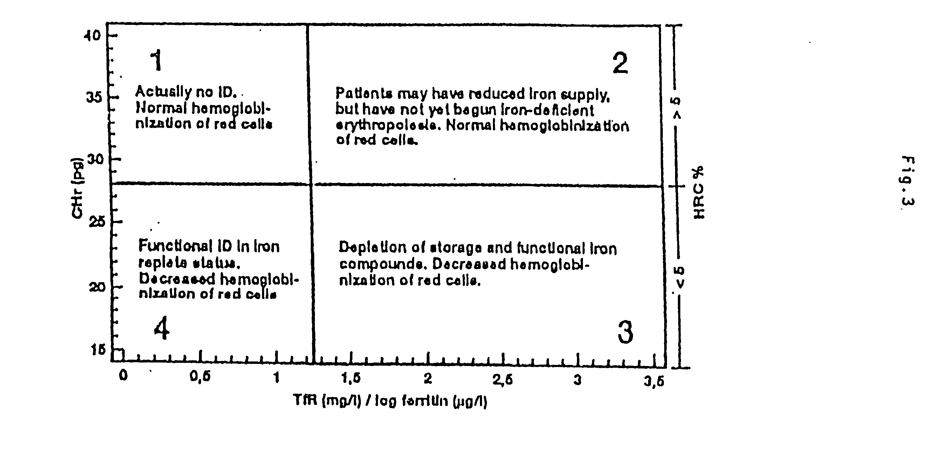

N group (non-anaemic group without APR) The control group consisted of 71 patients which were found in quadrant 1 (left top, fig. 3) in the diagnostic blots comprising 4 quadrants.

A group (anaemic group without APR) 79 anaemic patients without APR were examined and assigned to quadrant 2 (figure 3).

NA group (non-anaemic group with APR) This group consisted of 80 patients which were classified in quadrant 4 (figure 3).

AA group (anaemic group with APR) This group consisted of 143 patients which were classified in quadrant 3 (figure 3).

Patients with data points in quadrant 1 had a CHr of ≥ 28 pg.

Patients in quadrant 2 are iron-deficient according to the TfR-F index. All patients in this quadrant have a CAA and HRC > 5 %. The pattern CHr > 288 pg, HRC > 5 %, elevated TfR and normal or elevated ferritin indicated that these patients with CRA and APR have a reduced iron supply as indicated by the increase in TfR which, however, was not sufficient to cause a functional iron deficiency.

Patients with data points in quadrant 3 had the lowest ferritin and highest Tf concentrations. Tf is a negative acute phase reactant and the mean concentration was reduced in patients with an iron replete status in quadrants 1 and 4. In patients of quadrant 3 with haematological and biochemical identified iron deficiency, APR did not, however, cause a decrease in the serum Tf which indicates that the positive stimulus of iron deficiency is larger than the negative stimulus of APR on Tf synthesis.

The patients with data points in quadrant 4 had a CHr of < 28 pg and a HRC of > 5%.

In summary this means that the allocation of the data points to one of the quadrants 1 to 4 in the diagnostic plot denotes the following for the identification of iron deficiency in the diagram CHR against TfR/log ferritin:
Quadrant 1: no biochemical or haematologically identified iron deficiency
Quadrant 2: only biochemically identified iron deficiency
Quadrant 3: biochemically and haematologically identified iron deficiency
Quadrant 4: only haematologically identified iron deficiency.

The patient groups can be subdivided as follows according to the haematological and biochemical results:
Group N: non-anaemic, no APR; Hb (men) ≥ 140 g/l, Hb (women) ≥ 123 g/l, CRP ≤ 5 mg/l, WBC ≤ 10,000 /µl, ESR (erythrocyte sedimentation rate)≤ 30 mm/h, RDW (red cell distribution width) ≤ 15 %;
Group A: anaemic, no APR; Hb (men) < 140 g/l, Hb (women) < 123 g/I, CRP ≤ 5 mg/l, WBC ≤ 10,000/µl; ESR ≤ 30 mm/h;
Group NA: non-anaemic with APR; Hb (men) ≥ 140 g/l, Hb (women) ≥ 123 g/l, CRP > 5 mg/l or WBC > 10,000/µl or ESR > 30 mm/h or RDW > 15 %;
AA: anaemic with APR: Hb (men) < 140 g/l, Hb (women) < 123 g/l, CRP > 5 mg/l or WBC > 1 0,000/µl or ESR > 30 mm/h.

## Claims

1. In vitro method for determining the iron status and in particular for detecting disorders of iron metabolism comprising the determination of
(i) a parameter which allows a determination of the total body iron stores,
(ii) a parameter which allows a determination of the erythropoietic maturation process and/or its activity,
(iii) a parameter which allows a determination of unspecific disorders of iron metabolism, wherein the parameter is a biochemical parameter, and
(iv) a haematological parameter,
wherein ferritin is determined as parameter (i), soluble transferrin receptor (sTfR) is determined as parameter (ii), CRP is determined as parameter (iii), and MCH is determined as parameter (iv), wherein MCH is the average hemoglobin content of an erythrocyte cell,
**characterized in that**
differential diagnosis is carried out.

2. Method as claimed in claim 1,
**characterized in that**
the iron status and in particular disorders of iron metabolism are classified.

3. Method as claimed in claim 2,
**characterized in that**
the iron status is classified into one of the following groups:
(A) iron distribution disorders or/and iron utilization disorders with acute phase reaction,
(B) iron overloading,
(C) normal iron status, and
(D) deficiency of storage iron.

4. Method as claimed in claim 2 or 3,
**characterized in that**
the required treatment is recommended on the basis of the classification of the disorder of iron metabolism.

5. Method as claimed in claim 3,
**characterized in that**
an EPO therapy is indicated for classification in group (A), blood letting is indicated for a classification in group (B), no therapy is indicated for a classification in group (C) and iron substitution is indicated when classified in group (D).

6. Method as claimed in one of the previous claims,
**characterized in that**
the progress or/and response to treatment is observed or/and monitored.

## Patentansprüche

1. In-vitro-Verfahren zum Bestimmen des Eisenzustandes und insbesondere zum Nachweis von Eisenstoffwechselstörungen, umfassend die Bestimmung
(i) eines Parameters, der eine Bestimmung des Gesamtbestands an Körpereisen ermöglicht,
(ii) eines Parameters, der eine Bestimmung des erythropoetischen Reifungsprozesses und/oder dessen Aktivität ermöglicht,
(iii) eines Parameters, der eine Bestimmung von unspezifischen Störungen des Eisenstoffwechsels ermöglicht, worin der Parameter ein biochemischer Parameter ist, und
(iv) eines hämatologischen Parameters,
worin Ferritin als Parameter (i) bestimmt wird, löslicher Transferrinrezeptor (sTfR) als Parameter (ii) bestimmt wird, CRP als Parameter (iii) bestimmt wird und MCH als Parameter (iv) bestimmt wird, worin MCH der mittlere Hämoglobingehalt einer Erythrozytenzelle ist
**dadurch gekennzeichnet, dass**
Differzialdiagnose durchgeführt wird.

2. Verfahren nach Anspruch 1,
**dadurch gekennzeichnet, dass**
eine Klassifizierung des Eisenzustandes und insbesondere von Eisenstoffwechseistörungen erfolgt.

3. Verfahren nach Anspruch 2,
**dadurch gekennzeichnet, dass**
der Eisenzustand in eine der folgenden Gruppen klassifiziert wird:
(A) Eisenverteilungsstörungen oder/und Eisenverwertungsstörungen mit Akut-Phase-Reaktion,
(B) Eisenüberladung,
(C) normaler Eisenstatus und
(D) Mangel an Depoteisen.

4. Verfahren nach Anspruch 2 oder 3,
**dadurch gekennzeichnet, dass**
die Empfehlung zur benötigten Behandlung auf Basis der Klassifizierung der Eisenstoffwechselstörung erfolgt.

5. Verfahren nach Anspruch 3,
**dadurch gekennzeichnet, dass**
eine EPO-Therapie indiziert ist für eine Klassifizierung in Gruppe (A), Aderlass indiziert ist für eine Klassifizierung in Gruppe (B), keine Therapie indiziert ist für eine Klassifizierung in Gruppe (C) und Eisensubstitution induziert ist bei Klassifizierung in Gruppe (D).

6. Verfahren nach einem der vorhergehenden Ansprüche,
**dadurch gekennzeichnet, dass**
der Fortschritt oder/und das Ansprechen auf die Behandlung beobachtet oder/und kontrolliert wird.

## Revendications

1. Procédé *in vitro* pour déterminer le statut en fer et en particulier, pour détecter des troubles du métabolisme du fer, comprenant la détermination
(i) d'un paramètre qui permet une détermination des réserves corporelles totales en fer
(ii) d'un paramètre qui permet une détermination du processus de maturation érythropoïétique et/ou son activité et
(iii) d'un paramètre qui permet une détermination de troubles non spécifiques du métabolisme du fer, le paramètre étant un paramètre biochimique, et
(iv) d'un paramètre hématologique,
dans lequel la ferritine est déterminée comme paramètre (i), le récepteur de la transferrine soluble (sTfR) est déterminé comme paramètre (ii), la CRP est déterminée comme paramètre (iii) et la MCH est déterminée comme paramètre (iv), la MCH étant la teneur moyenne en hémoglobine d'une cellule érythrocytaire, **caractérisé en ce**
**qu'**un diagnostic différentiel est réalisé.

2. Procédé selon la revendication 1,
**caractérisé en ce que**
l'on classe le statut en fer et en particulier des troubles du métabolisme du fer.

3. Procédé selon la revendication 2,
**caractérisé en ce que**
l'on classe le statut en fer dans l'un des groupes suivants :
(A) troubles de la distribution du fer et/ou troubles de l'utilisation du fer avec une réaction de phase aiguë,
(B) surcharge en fer
(C) statut en fer normal, et
(D) insuffisance en fer de réserve.

4. Procédé selon la revendication 2 ou 3,
**caractérisé en ce que**
le traitement nécessaire est recommandé sur la base du classement du trouble du métabolisme du fer.

5. Procédé selon la revendication 3,
**caractérisé en ce**
**qu'**une thérapie avec de l'EPO est indiquée pour le classement dans le groupe (A), une saignée est indiquée pour le classement dans le groupe (B), aucune thérapie n'est indiquée pour le classement dans le groupe (C) et un remplacement du fer est indiqué lors du classement dans le groupe (D).

6. Procédé selon l'une des revendications précédentes,
**caractérisé en ce que**
l'on observe et/ou on surveille l'évolution et/ou la réponse au traitement.
